(19) Europäisches Patentamt

European Patent Office

Office européen des brevets

(11) **EP 1 249 207 B1**

(12) **EUROPEAN PATENT SPECIFICATION**

(45) Date of publication and mention
of the grant of the patent:
**04.01.2006 Bulletin 2006/01**

(51) Int Cl.:
*A61B 6/12* (2006.01)     *A61B 19/00* (2006.01)
*G01R 33/58* (2006.01)     *G01N 23/04* (2006.01)

(21) Application number: **02077343.8**

(22) Date of filing: **10.02.1994**

(54) **Fiducial marker**

Markierung als Bezugspunkt

Repère de calage

(84) Designated Contracting States:
**BE CH DE FR LI NL SE**

(30) Priority: **12.02.1993 US 17167**

(43) Date of publication of application:
**16.10.2002 Bulletin 2002/42**

(62) Document number(s) of the earlier application(s) in
accordance with Art. 76 EPC:
**94909815.6 / 0 684 786**

(73) Proprietor: **Allen, George S.**
**Nashville, TN 37205 (US)**

(72) Inventors:
• **Jandak, Jennifer J.**
**Sausalito, California 94965 (US)**

• **Fitzpatrick, Michael J.**
**Nashville, TN 37205 (US)**
• **Willcott, Robert M.**
**Nashville, TN 37215 (US)**
• **Maciunas, Robert J.**
**Ohio 44022 (US)**
• **Maurer, Calvin R., Jr.**
**Nashville, TN 37212 (US)**
• **Allen, George S.**
**Nashville, TN 37205 (US)**

(74) Representative: **Ede, Eric**
**Fitzpatricks**
**1 Blythswood Square**
**Glasgow G2 4AD (GB)**

(56) References cited:
**EP-A- 0 146 699**     **US-A- 4 618 978**
**US-A- 4 991 579**     **US-A- 5 178 146**

EP 1 249 207 B1

## Description

Background of the Invention

Recent years have seen the development of diagnostic techniques that allow the practicing clinician to obtain high fidelity views of the anatomical structure of the human body. Imaging systems such as computed tomographic (CT) x-ray imagers, positron emission tomographic (PET) scanners, single photon emission computed tomography (SPECT) scanners and nuclear magnetic resonance imaging (MRI) machines have provided clinicians with the ability to improve visualization of the anatomical structure of the human body without surgery or other invasive techniques. In lieu of exploratory surgery, the patient can be subjected to the scanning modalities of such imaging systems, and the patient's anatomical structure can be reproduced in a form for evaluation by a trained doctor.

The doctor sufficiently experienced in these techniques can evaluate the images of the patient's anatomy and determine if there are any abnormalities present. An abnormality in the form of a lesion appears on the image as a shape that has a discernable contrast with the surrounding area. The difference in contrast is due to the lesion having imaging properties that differ from those of the surrounding body tissue. Moreover, the contrasting shape that represents the lesion appears at a location on the image where such a shape would not normally appear with regard to a similar image of a healthy person.

Once a lesion has been identified, several methods of treatment are utilized to remove or destroy the lesion, including chemotherapy, radiation therapy, and surgery. When chemotherapy is chosen, drugs are introduced into the patient's body to destroy the lesion. During the course of treatment, imagers are commonly used to follow the progress of treatment by subjecting the patient to periodic scans and comparing the images taken over the course of the treatment to ascertain any changes in the lesion configurations.

For example EP0146699 describes implants consisting of a contrast body and fastener body which are locally fixed into bone with the aid of a structure on the fastener body and thus form stationary reference points in the bone for the measurement of x-ray images.

In radiation therapy, the images of the lesion generated by the imager are used by a radiologist to adjust the irradiating device and to direct radiation solely at the lesion while minimizing or eliminating adverse effects to surrounding healthy tissue. During the course of the radiation treatment,-the imaging system is also used to follow the progress of the patient in the same manner described above with respect to chemotherapy.

When surgery is used to remove a lesion or other abnormality, the images of the lesion in the patient can guide the surgeon during the operation. By reviewing the images prior to surgery, the surgeon can decide the best strategy for reaching and biopsying, excising, or otherwise manipulating the abnormality or lesion, whether it is a brain tumor, arteriovenous malformation, infection or other entity. After surgery has been performed, further scanning is utilized to evaluate the success of the surgery and the subsequent progress of the patient.

A problem associated with the scanning techniques mentioned above concerns the accurate selection and comparison of views of identical areas in images that have been obtained by imagers at different times or by images obtained essentially at the same time using different image modalities, e.g., CT, MRI, SPECT, and PET. This problem has two aspects. First, in order to relate the information in an image of the anatomy to the anatomy itself, it is necessary to establish a one-to-one mapping between points in the image and points on the anatomy. This is referred to as registering image space to physical space.

The second aspect concerns the registration of one image space to another image space. The goal of registering two arbitrarily oriented three dimensional images is to align the coordinate systems of the two images such that any given point in the scanned anatomy is assigned identical addresses in both images. The calculation of the rigid body transformation necessary to register the two coordinate systems requires knowledge of the coordinate vectors of at least three points in the two systems. Such points are called "fiducial points" or "fiducials," and the fiducials used are the geometric centers of markers, which are called "fiducial markers". These fiducials are used to correlate image space to physical space and to correlate one image space to another image space. The fiducial markers provide a constant frame of reference visible in a given imaging mode to make registration possible. The general technique for using fiducial markers to obtain registration of image data across time is set forth in U.S. Patent No. 4,991,579 to George S. Allen.

One problem extant in the field lies in the provision of fiducials capable of use with several imaging modalities. MRI and X-ray CT images are digital images, in which the images are formed point by point. These points are called picture elements, or pixels, and are associated with an intensity of light emitted from a cathode ray tube, or are used to form an image on film. The array of lighted pixels enables the observer to view an image. The manner in which the intensity of any given pixel is altered or modulated varies with the imaging modality employed. In X-ray CT, such modulation is a function primarily of the number of electrons per unit volume being scanned. In MR imaging, the parameters primarily influencing this modulation are the proton spin density and longitudinal and transverse relaxation times T1 and T2, which are also known as the spin-lattice and spin-spin relaxation times, respectively. In constructing a fiducial marker, one must be aware that an agent that can be imaged under one imaging modality will not necessarily be imageable under another modality. And yet, the ability to image under both CT and MRI with a given marker would be especially useful, in that one would then be able to register images derived from different imaging modalities. For example, the capability to register CT and MR images would allow the integration of information concerning bony structure provided by a CT scan with the soft tissue anatomical information provided by an MRI scan. There remains a need for a fiducial marker that can be used to establish a known coordinate system under several imaging modalities.

A further problem in the field arises from the competing needs of accommodating patient comfort, which would tend to lead clinicians toward the minimization of marker size, with the desire of clinicians to use markers that are as bright and thus as large as possible. Such brightness is desirable because it provides a strong signal that can be distinguished from noise inherent in the imaging process. The use of large-sized markers is also desirable so that the image of the marker occupies as many pixels as possible. Increasing the number of pixels occupied by the marker increases the accuracy with which the position of the marker can be determined. Furthermore, the general technique of using fiducial markers requires the determination of the centroid of the marker; it is easier to compute the centroid for a large, bright marker than for a smaller, dimmer marker. On the other hand, the larger the marker is, the more difficult it is for the patient to tolerate its presence for extended periods of time. There remains a need for a marker which can exploit the advantages presented by increased size that would also be tolerated by the patient during the period of its use. There is also a need for a small multi-modality marker that can be implanted into a patient and remain there for more extended periods of time.. Such a more permanent fiducial marker would preferably be detectable by a non-invasive technique so that its position in physical space could be determined and its centroid computed even as it remained hidden from visual inspection beneath the patient's skin.

US 5178146 describes a grid system for interfacing MRI with other imaging modalities. The system includes a grid of contrast material which is compatible with MRI and other imaging modalities and a means to reproduceably position the subject in relation to the grid. The grid system is also used to plan radiotherapy and surgical biopsy procedures. Such a system is complex and can lead to inaccuracy.

Summary of the Invention

In view of the foregoing needs, the present invention provides medical workers with fiducial markers that can be imaged under a variety of imaging modalities, i.e., are multi-modal. The markers can be used to register image space onto image space across imaging modalities. The fiducial marker may also be used for the registration of imaging space and physical space for the successful performance of image guided craniotomies, biopsies, cyst aspirations, radiation therapy, ventricular shunt placements, and other similar surgeries.

A fiducial marker having features of the present invention includes a hollow container, preferably cylindrical or spherical, that may be made of imageable material and which is filled with liquids suitable for various imaging modalities. In one version, the marker is of suitably compact size and shape to be implanted into bone for periods of prolonged duration that may be measured in years. A cylindrical shape is preferred, as it minimizes the size of the incision required for insertion by maximizing the available volume of contrast agent for a given incision size. A permanently implanted marker allows comparison of scans over time for follow-up therapy (for example, to make lesion volume comparisons in order to monitor growth). It also allows fractionated radiotherapy, in which small doses of radiation are administered frequently over the course of treatment.

One known measure for defining known set of points about the human skull for this purpose involves the use of a stereotactic frame (see U.S. Patent No. 4,608,977 for a general description of such a device). At present, a stereotactic frame cannot be used for this therapy because the frame poses a significant risk of infection, is too painful, or is too bulky and restrictive to be left on for an extended time and cannot be re-attached in the same location to tolerance of submillimetric accuracy. This problem is resolved by the use of an implantable marker that can be well tolerated by the patient for extended periods of time. Such an implantable fiducial marker can also be localized in the radiation therapy suite and thereby enable the patient's image space, intracranial physical space, and radiation therapy device to be registered with each other.

In another embodiment, the fiducial marker takes the form of a relatively larger temporary marker that is removably attached to a base that is rigidly affixed to bone. In this embodiment, the base portion is left in place for a period of days or weeks and is provided with means for detachably receiving an imaging marker. By permitting the removal of the imaging marker after scanning, the over-all height profile of the subcutaneous marker base is reduced, adding to its overall stability during implantation. In this way, the imaging marker, which need only be kept in place attached to its base for the few hours required for the medical procedure, can be made larger than could otherwise be tolerated in the case of markers left in place for days on end. The larger imaging marker produces a brighter image and is easier to localize in image space than would be the case for a smaller marker.

The interchangeable nature of this marker also makes it suitable for use with PET and/or SPECT scans. In both of these modalities, an image marker must be radioactive. Furthermore, in both of these modalities, it is necessary to obtain with each imaging scan a so-called "transmission scan," in which no radioactive substance is present. The transmission scan must be obtained with the patient in the same position as the imaging scan, and it is therefore not feasible for the marker bases to be implanted between scans. Instead, it is necessary to attach a nonradioactive marker for the transmission scan and then to replace the nonradioactive marker with a radioactive one for the imaging scan. The visibility of the markers in CT, MRI, PET, and SPECT images allows one to register images obtained with any of these modalities.

In both the temporary and the more permanent versions of the invention, the container is charged with aqueous imaging agents to provide imaging capability in MRI. CT imaging capability is provided by mixing the aqueous MRI imaging agents with other aqueous agents imageable under CT and optionally, by doping the plastic housing with agents that will render the marker housing imageable under CT, in which case the shape of the housing so doped is such that its volume centroid is coincident with the center of the volume occupied by the MRI imaging agent. The imaging agents are selected so as to provide suitable imaging in both modalities and, where an aqueous CT imaging agent is employed, must be miscible. The use of a miscible liquid combination results in the same volume being visible in different imaging modalities with coincident centers for the purpose of locating the center of the marker. In the embodiment for PET and/or SPECT, and external marker is filled with the appropriate radioisotope and used in place of the MRI/CT markers described in the previous paragraph. In an alternative embodiment employing the temporary fiducial marker, a kit of markers can be provided in which each marker is optimized for one imaging modality (MRI, CT, PET, or SPECT).

Various embodiments of the invention are set forth in the appendent claims.

Brief Description of the Drawings

For a more complete understanding of this invention, reference should now be made to the embodiments illustrated in greater detail in the accompanying drawings and described below. In the drawings:

FIG. 1A is an exploded perspective view of a temporary fiducial marker embodying features of the present invention, in which the imaging marker is shown separated from the base with respect to which it can releasably be attached;
FIG. 1B shows the marker of FIG. Ia as assembled;
FIG. 2A is an elevational view of the fiducial marker assembly, in which the imaging marker is shown attached to the base;
FIG. 2B illustrates in cross section the invention shown in FIG. 2A as viewed along line A-A;
FIG. 3A is an elevational view of the base portion of the invention;
FIG. 3B is a view of the top portion of the base ;
FIG. 3C is a cross sectional view of the base taken along line C-C showing the grooves that receive the imaging marker;
FIG. 4A is an elevational view of the cap portion of the imaging marker;
FIG. 4B is a top plan view of the cap portion shown in FIG. 4A;
FIG. 4C is a bottom plan view of the cap: and
FIG. 5 is a cross sectional view of a different version of the invention.

Detailed Description

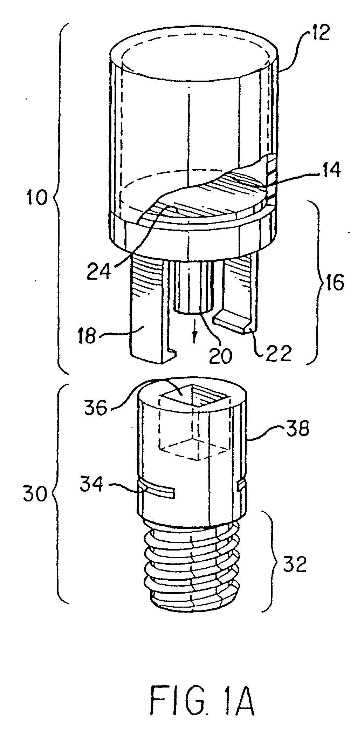

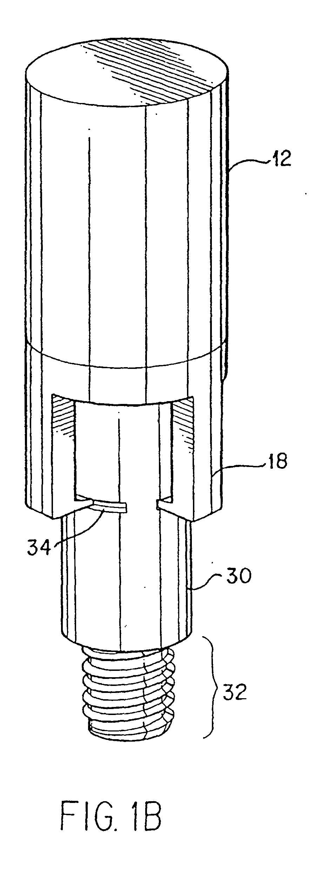

Referring now specifically to the drawings, wherein like numerals indicate like parts throughout, a version of a temporary fiducial marker assembly is indicated in FIGS. 1 - 4. These figures illustrate a fiducial marker assembly comprising an imaging marker 10 and a base 30.

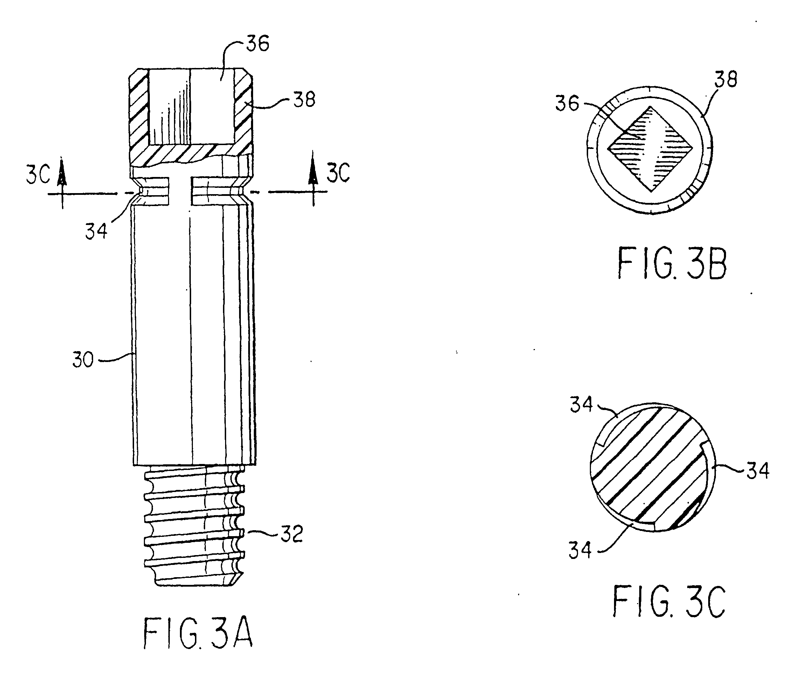

The base 30 has a threaded portion 32 at a first end. The threads enable a surgeon to securely attach the base into the skull or other desired portion of bone tissue. Other connecting structure is provided to securely and releasably link the imaging marker with the base. For example, in the illustrated embodiment, the end of the base opposite the threaded portion terminates in a socket head 38 which contains a socket-like recess 36. (It is anticipated that the base will be implanted into bone with the aid of an insertion tool that twists the base into the bone or into a hole provided in the bone. The recess is non-circular so as to better transmit the torque provided by such an insertion tool.) Just beneath the socket head 38 are a plurality (as seen in FIG. 3C, three) of grooves 34. As shall be further explained below, the socket 38 and the grooves 34 provide for the secure and releasable attachment of the imaging marker portion with base portion.

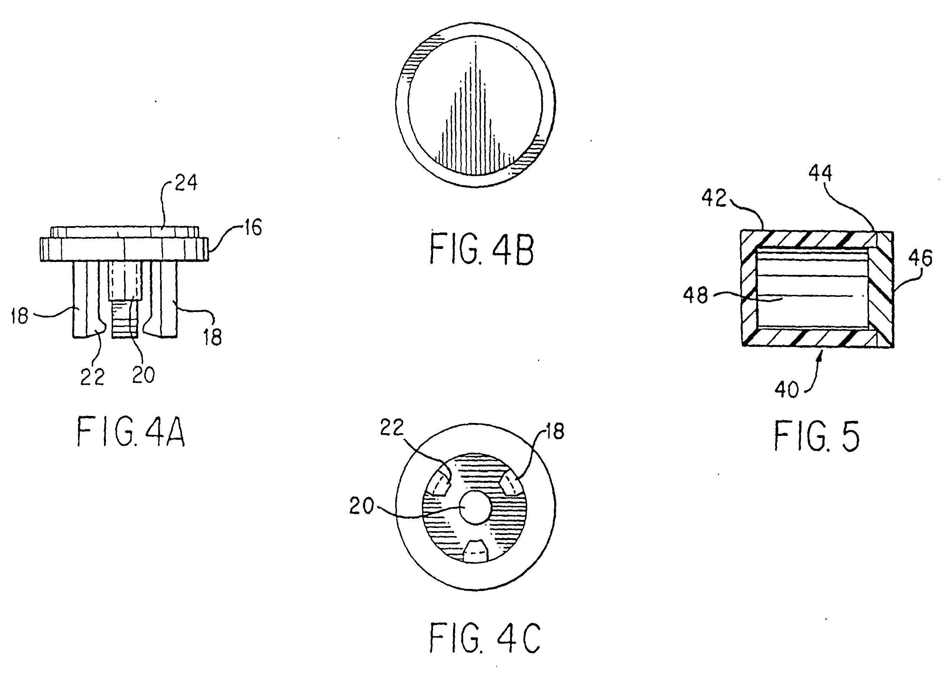

The imaging marker portion of the temporary fiducial marker assembly may consist of two principal portions, a cylinder 12 and a cap 16 (see FIGS. 4A - 4C). The cylinder 12 contains a cavity 14 for receiving a mixture of imaging agents whose composition is determined by the imaging modalities to be employed. While in this version, the vessel containing the imaging agents is preferably cylindrical so as to simplify the process by which the centroid of the corresponding volume of imaging agent is determined, other shapes (such as a box or sphere) could be employed as well. The cylinder 12 is closed at one end and open at the other to allow for the introduction of the imaging agents. In one version of the device, a cap 16 is used to seal off the open end of the cylinder once the imaging agents have been added to the cylinder. In this version, the cap may be cemented or welded into place. The cap may be provided with a plug portion 24 that protrudes into and thereby helps seal off the cavity 14 of the cylinder 12 against leakage of the imaging agents. Other conventional filling and sealing techniques, such as blow-molding, ultrasonic welding, or heat sealing, may be used.

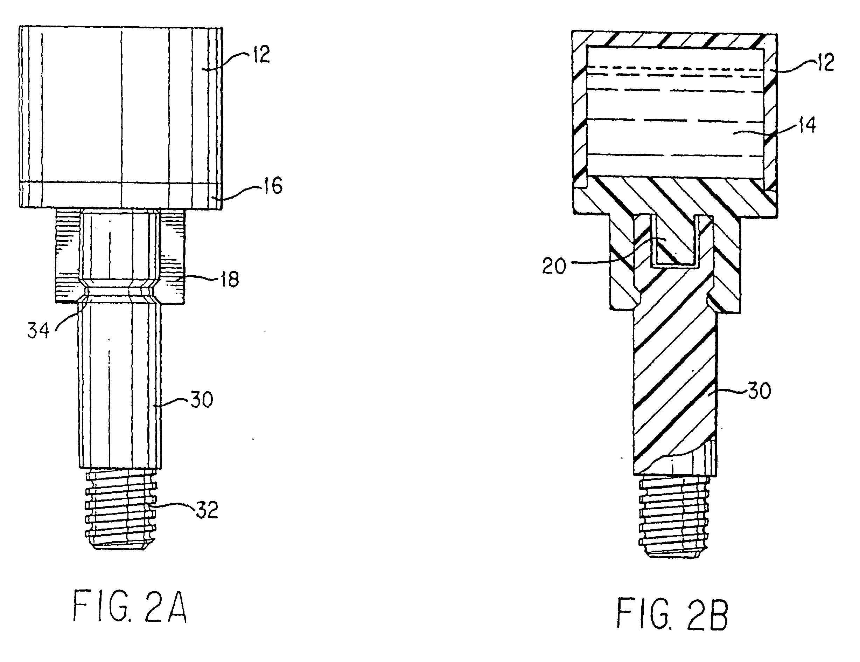

Where a cap is employed, it may be provided with a protruding boss 20 and a plurality (here, three) of snap arms 18, which terminate with inwardly projecting portions 22. The shape and dimensions of the boss are in direct correspondence with the shape and size of the socket 36 provided in the base 30 so as to properly and securely center the imaging marker on the base. The snap arms 18 cooperate with the grooves 34 of the base 30 so as to detachably secure the imaging marker onto the base. The cooperation of these elements is illustrated in FIGS. 2a and 2b. While this example shows the use of snap arms, other fastener structure may be provided for attaching the marker to the base (e.g., screw threads, clasps, hooks, etc.).

The dimensions of the temporary fiducial marker assembly will be somewhat dependent on the state of the art of imaging. The greater the sensitivity of the scanner employed, the lesser the quantity of imaging material necessary to provide a suitable image, which in turn makes it possible to reduce the corresponding size of the marker that must be employed to contain the imaging material. The Applicants have found that a base portion approximately 12mm in length and 2mm-3mm in diameter is sufficiently large to provide for the secure placement of the base into the bone beneath the skin. When the clinician prepares the patient for imaging, the base portion is exposed and an imaging marker approximately 6mm in length is attached to the base; the marker itself may protrude from the scalp and be exposed to air while a scan is performed on the patient. The base and the imaging marker housing are constructed of a bio-compatible organic polymer, such as polyether imide.

FIG. 5 illustrates a second embodiment of the fiducial marker, which may be left implanted entirely beneath the skin for extended periods of time. The marker comprises a cylinder 42 defining a space into which are placed the desired imaging agents. As noted in the Summary, a cylindrical shape is preferred, because this shape minimizes the size of the incision that must be made for the marker s insertion. It is also the shape that best corresponds to the hole that one drills in the bone to accommodate the marker. The body of the cylinder is sealed off with a cap 46 or is otherwise sealed. The body is preferably constructed of an organic polymer known to be well tolerated by the body for extended periods of time, such as polymethyl methacrylate, high density polyethylene, or ceramics such as zirconium oxide and aluminum oxide. The entire marker assembly is small enough for long-term implantation into bone without causing distortion of the bone over time. One exemplary size provides for the marker to be 4 mm in length and 3 mm in diameter.

As shall be explained below, the judicious choice of aqueous imaging agents allows for the construction of a marker that is visible under both CT and MRI imaging modalities. Furthermore, by using a marker that comprises a solid outer portion and an aqueous inner portion, the marker can be located through the use of a non-invasive transcutaneous detection system, such as one employing ultrasound to detect the presence of the solid-liquid interface between the aqueous core and the solid outer portion.

Because the fiducial marker assembly is to be used in a variety of imaging modalities, the use of solid metal is eschewed throughout. The presence of metal may cause unwanted artifacts and image distortion in the image, and may impede efforts to localize the marker (i.e., locate and identify its centroid). The properties characteristic of solid metal, such as high electrical conductivity, paramagnetism, and, for some metals, ferromagnetism, are generally inappropriate for use in MRI.

Although metals have been used in the past as CT markers, they are often generally less than optimal because the high linear attenuation of metals may cause unwanted image artifacts, such as starbursts. While these artifacts can be reduced somewhat by reducing the size of the markers, this reduction in size also reduces the accuracy with which the location of the marker can be determined. In MRI, metals cause disturbances in the local magnetic field (the so-called "susceptibility" artifact) which diminish the image intensity and physically shift the position of the image. Such physical phenomena are unsuitable in a fiducial marker. The choice of materials selected as imaging agents is therefore driven by the physics underlying the image modalities that are employed.

In CT studies of human tissue, the brightest anatomical features imaged are bone, which attenuates X-rays more strongly than other tissue. This attenuation is characterized by the so-called "linear attenuation coefficient," which measures the X-ray attenuation per unit of path length. The linear attenuation coefficient increases with increasing electron density (the number of electrons per unit of volume). In order for an X-ray CT imager to produce pixels brighter than bone when imaging a material (such as that of a marker), the material being scanned must have an electron density that is greater than that of bone. Therefore, one approach to enhancing the absorption of X-rays is to increase the electron density per unit volume. This can be accomplished by adding compounds having atoms of high atomic number (Z) to the imaged object, or by substantially increasing the density of the material being scanned. Any high Z value atom suffices. Suitable materials include barium, iodine, titanium, tantalum, silver, gold, platinum, and stainless steel. These materials may be solid, or dissolved as ions in biologically compatible fluids. However, as noted above, the use of solid metal in a fiducial marker tends to create artifacts that degrade the ability of the marker to be used as a true fiducial. These artifacts are higher for markers that have higher linear attenuation coefficients. They are also higher for markers of larger sizes. In particular, for a given linear attenuation coefficient, if the shape and size of the marker is altered so that there is a longer path length through the marker, the tendency to cause an image artifact increases. It is therefore necessary to provide the high Z value material in a diluted form.

One approach to providing an imageable marker of appropriate size that does not yield unwanted artifacts is to dope the marker housing with a CT imaging agent. For example, the housing for the marker, which is normally made of organic polymers, can have barium added as a salt. Alternatively, titanium dioxide may be added to the polymeric housing. Salts of gold or of platinum are also effective materials for rendering the housing shell radio-opaque and thus imageable under CT scanning. Concentrations of these metal salts of up to about 400 mg/ml can be used in the markers without causing appreciable image artifacts. It must be born in mind that it will still be necessary to locate the geometric center of the marker housing so doped; therefore, the geometry of the doped housing should preferably be configured so that its center will be coincident with the center of the volume of any other imaging agent used to accommodate other imaging modalities.

Another approach to providing high Z value agents without resorting to the use of solid metal is to provide them in the form of an aqueous solution. Aqueous solutions of compounds having high Z atoms, such as barium, iodine, titanium, tantalum, silver, platinum, and iron can be used as imaging agents in the fiducial marker of this invention. In particular, compounds of iodine and silver have been found to be effective for this purpose.

For example, in certain preferred embodiments, an aqueous solution of an iodine containing organic molecule with an effective iodine concentration of between about 50 to about 600 mg/ml provides an effective CT imaging agent. In another preferred embodiment, silver nitrate dissolved in water at concentrations of between about 100 mg/ml to about 600 mg/ml is effective. Either solution will be effective in CT at imaging the volume defined by the marker cavity.

By judicious choice of a high-Z aqueous solution, it is possible to create a set of markers having absorption properties in X-ray CT that are anywhere between those of water and 15 times those of bone. However, the effect of these agents on MRI imaging must also be considered, as must the additional requirements that MRI imaging presents.

For example, the use of aqueous solutions where only CT imaging is contemplated is optional - other carriers, such as oils, could be used as carriers for high Z-number elements. However, this is not the case with respect to MRI. The accurate location of MRI markers in biological tissue must be done with an aqueous imaging agent. Substances other than water exhibit different resonance frequencies, or chemical shifts, and will appear in the image displaced from their true location. Therefore, where a common liquid carrier is to be used both for CT as well as MRI imaging agents, it should be water. (The phenomenon of chemical shift is further described in the literature and is well known to practitioners in the art - see e.g., "Edge Artifacts in MR Images: Chemical Shift Effect," Journal of Computer Assisted Tomography 9(2):252-257 (1985).)

The physics of MRI scanning must further be considered. Pure water is characterized in proton nuclear magnetic resonance by a high spin density and long T1 and T2 values. The relaxation times are in the range of two to four seconds. MRI images are partly based on spin density as well as T1 and T2. The effect of spin density on image brightness is linear; reducing the spin density by one half reduces the brightness by one half. The effects of T1 and T2 are exponential; for example, altering these by one half results in a change of 86% in the brightness in the image. These factors may be summarized mathematically as follows:

$$I = NC(e^{-(TE/T2)})(1-e^{-(TR/T1)})$$

where I = signal intensity, N = spin density, the parameters TR and TE are the repetition time and the echo time determined by the radio frequency and gradient pulses employed, and C is a constant of proportionality that depends on the scanner and the pulses employed.

These three parameters, T1, T2, and spin density, can be altered by the addition of chemicals selected for their physical properties in solution. One such property is paramagnetism, in which the added material has an unpaired electron in its electron configuration. Such agents shorten the relaxation times T1 and T2 drastically. Another such property is viscosity, other chemicals may be added to the solution for their ability to alter the viscosity of a solution, even to a point of making a gel. Viscosity is an important consideration, because it is inversely correlated with T2. The more viscous a solution, the greater the number of bonds that are present and the less able the hydrogen nuclei are to react and respond to the magnetic field, which results in a dimmer image. These viscosity enhancing agents dilute the water in the solution, thereby reducing the spin density, and reduce the relaxation times $T_1$ and $T_2$. Whatever the added material, T1 will always be equal to or greater than T2.

Because of the small concentrations of MRI imaging agents appropriate for MRI enhancements, these agents have no significant effect on the CT imaging agent. However, the efficacy of an MRI imaging agent may be obscured by the presence of a CT imaging agent. The MR image is modulated by the cumulative effects of all solutes added to an aqueous medium. This modulation is due to interactions with the hydrogen atoms in water which provide the MR signal. The general effects of three typical CT imaging agents, of one typical MR imaging agent, and of selected combinations of these two types of agents are presented below.

In accordance with the above discussion, it has been determined that silver nitrate, which, as noted above, provides a suitable image under CT scans when provided in concentrations up to about 600 mg/ml, reduces the observed spin density (and hence reduces the brightness of the MR image) by up to 50%. At a preferred concentration of about 600 mg/ml, the observed T1 and T2 values are about 1 second. As the concentration of silver nitrate decreases, the T1 and T2 values approach those of pure water. This observed effect affords the opportunity to provide a variety of marker compositions based on silver nitrate (as the CT agent) to create differing contrast levels with respect to human tissue under MRI.

A solution of iohexol (a non-ionic X-ray CT imaging agent) in concentrations of up to about 600 mg/ml of iodine also reduces the observed spin density by up to 50%. At a concentration of 150 mg of iodine per ml of aqueous solution, the observed T1 value is 300-400 milliseconds, and the observed T2 value is 100-120 milliseconds. The effect on these parameters is in accord with similar effects noted from increasing the viscosity of aqueous solutions. Over the entire range of iohexol concentrations, it has been determined that one can alter the spin density by 50%, change the T1 value from 4 seconds to 0.15 seconds, and the T2 value from 4 seconds to 0.04 seconds. Thus, iohexol too, may be used to provide a variety of marker compositions to create different contrast levels with respect to human tissue under MRI.

A solution of iothalamate meglumine (an ionic X-ray CT imaging agent) in concentrations up to about 600 mg/ml of iodine reduces the observed spin density by factors ranging up to 75%. At a concentration of 175 mg of iodine per ml of aqueous solution, the observed T1 value is 1200-1500 milliseconds, and the observed T2 value is 300-350 milliseconds. The solution is not viscous at this concentration. Over the entire range of iothalamate meglumine concentrations it has been determined that one can alter proton spin by 75%, change T1 value from 4 seconds to 1 second, and the T2 value from 4 seconds to 200 milliseconds. Thus iothalamate meglumine may be used to provide a variety of marker compositions to create different contrast with respect to human tissue.

Within limits, one can prepare solutions of reduced spin density in which T1 is equal to T2, and solutions providing spin density in which T1 is greater that T2. $T_1$ and T2 values of these altered solutions range from 1 millisecond to 4 seconds, while the spin density ranges from 0 molar (no water at all) to 111 molar in hydrogen (pure water). The instant invention identifies a number of substances suitable for use in MRI imaging in fiducial markers. One preferred suitable MRI imaging agent is gadopentetate dimeglumine. Another possible MRI imaging agent is gadoteridol. (Each of the aforementioned substances has received FDA approval for use as an injectable MRI imaging agent.) Other possible agents for use as MRI imaging agents include Ferric Chloride ($FeCl_3$) and Copper Sulfate ($CuSO_4$) in concentrations of between 0.5 mM and 5 mM. For a more complete listing of contrast agents and their properties, see chapter 14 of Magnetic Resonance Imaging, 2nd ed., edited by Stark and Bradley, 1992.

A solution of gadopentetate dimeglumine-DPTA (an injectable MR contrast agent) in concentrations up to about 0.5 mM (millimolar) is seen to have little effect on the observed spin density of the solution. At a concentration of 0.5 mM, the observed T1 value is 50 to 100 milliseconds, and the observed T2 value is 8 to 15 milliseconds, over the entire range of concentrations one observes spin density of 111 molar in hydrogen (water). T1 varies from 50 milliseconds to 4 seconds, and T2 varies from 8 milliseconds to 4 seconds. This is in accord with the reported effect of the paramagnetic material. By varying the marker compositions based on the gadopentetate compound, it is possible to create a variety of different contrasts with respect to human tissue.

The four solutions just discussed were based on one solute in water. To provide a multi-modality marker, binary mixtures of CT and MR contrast agents are considered. It has been observed that binary combinations of gadopentetate-DPTA with any of the other chemical compounds set forth produce a synergistic effect. The matrix of possibilities for altering spin density, T1 and T2, by varying the concentration of MRI and CT imaging agents, enhances the ability to tailor solutions to give maximum contrast in all three types of MR image parameters. For example, with respect to the permanent marker, iothalamate meglumine at a concentration of 175 mg/ml combined with gadopentetate meglumine-DPTA at 0.5 mM creates a solution with the following MR properties: a spin density corresponding to 75-80 molar water, T1 value of 400-500 milliseconds, and a T2 value of 150-200 milliseconds. A variety of other binary compositions permit one to create many different ratios of spin density, T1 and T2 values.

Hence, as these examples suggest, it is possible to create a set of MR markers with any combination of spin density, T1 and T2 that are smaller in magnitude than those of water. The optimum values of these parameters as specified by the marker application dictate the composition of the solution.

For one marker to be optimized for both MRI and CT, the imaging agents and their concentrations must be selected such that the solution and/or housing may be differentiated in the X-ray by its radio-opacity and at the same time be differentiated in the MRI by its MRI parameter set - spin density, T1, and T2. As noted above, it is known that aqueous solutions of compounds based on high Z number elements will provide the necessary degree of radio-opacity for CT imaging. It is also known that such substances may reduce the imaging efficacy of compounds selected for their use as MRI contrast agent, such as gadopentetate dimeglumine-DPTA. In order to combine the two agents in an effective manner, they are mixed together in varying concentration and tested under both CT and MRI scans until one has empirically determined a concentration of each that provides a marker that is acceptably imageable under both modalities.

As a result of such a course of testing, the Applicants have identified two preferred binary mixtures that meet these requirements when used in the permanent marker; these are iothalamate meglumine (175 mg of iodine/ml) with gadopentetate dimeglunine-DPTA (0.5 mM) and silver nitrate (350 mg/ml) with gadopentetate dimeglumine-DPTA (0.5 mM). In the case of the temporary marker, the concentrations of the CT imaging agents are reduced to 165 mg of iodine per ml of aqueous solution of iothalamate meglumine and 200 mg/ml of silver nitrate respectively.

The present invention may be embodied in other specific forms without departing from its essential characteristics. The imaging agents could be provided as mixtures of three or more compounds selected to optimize particular imaging characteristics.

The present invention may be embodied in other specific forms without departing from its essential characteristics. For example, a flatter, more disk-like marker than that which is shown in the figures may be employed. Increasing the largest dimension of X-ray traversal boosts the brightness of the image in CT. It also allows one to take CT scans with thicker slices in which the image of the marker does not become lost in the corresponding pixel. This is especially useful in trauma cases, when there is not sufficient time for more refined views.

In another variant, the imaging agents discussed above can be relied upon for one imaging mode and another technique relied on for locating the marker in a second imaging mode. For example, one may image a marker under a first modality (e.g., MRI) using the imaging agents discussed above, and then locate the marker in the image space generated by a second imaging modality (e.g., CT) by physically locating the marker in the physical space of the second imaging machine. For example, robotic arms such as are disclosed in U.S. Patent No. 5,142,930 or U.S. Patent No. 4,991,579 could be used for this purpose, since in the course of a scan the addresses of each point in image space are generally defined with respect to the imaging machine and hence to any arm (such as that disclosed in the '930 patent) or other device whose location is clearly defined with respect to that machine. One may then label the corresponding point in image space as containing the location of marker, even where it is not directly imageable in that imaging mode.

**Claims**

1. A fiducial marker assembly, including:

    an imaging marker (10) comprising imaging material and having a housing constructed of a bio-compatible material, the imaging marker (10) having a cavity (14) **characterised in that** the cavity (14) contains a mixture of aqueous imaging materials which enable the imaging marker (10) to be imageable under X-ray CT and MRI; the imaging materials being first and second aqueous solutions that are miscible to form a mixture within the cavity (14) wherein the first solution is radio-opaque and the second solution is imageable under nuclear magnetic resonance.

2. The device of claim 1, **characterised in that** it further comprises:

    a base (30) for supporting the imaging marker (10), said base (30) having means for accommodating the attachment of the base (30) to tissue;
    a connector for detachably securing the imaging marker (10) with the base (30); and
    means for sealing the cavity (14) of the imaging marker (10) against leakage of imaging material contained within the cavity (14).

3. The device of claim 2, **characterised in that** the means for sealing (24) the cavity (14) is a cap (16) that is securely connected to the housing of the imaging marker (10).

4. The device of claim 3, **characterised in that** the cap (16) includes a protruding boss (20) and the base includes a corresponding socket (36, 38) sized so that the boss (20) of the cap (16) can mate with the socket (36, 38) and thereby form a link between the cap (16) and the base (30).

5. The device of claims 2, 3 or 4, **characterised in that** the sealing means (24) is provided with a plurality of extending arms (18) for detachable attachment with the base (30).

6. The device of claims 2, 4 or 5, **characterised in that** the base (30) includes threads (32) for facilitating its attachment to tissue.

7. The device of claim 5, **characterised in that** the base (40) is provided with grooves (34) and the extending arms (18) of the sealing means (24) terminate in projecting portions (22) that cooperate with the grooves (34) on the base for connection therewith.

8. The device of any one of the preceding claims, **characterised in that** the marker housing is shaped so that its centroid of volume is coincident with the centroid of volume of the cavity defined therein.

9. The device of claim 8, **characterised in that** the marker housing is cylindrical in shape.

10. The device of claims 8 or 9, **characterised in that** the marker housing is formed of a polymer.

**11.** The device of claim 1, **characterised in that** the marker housing is doped with a radio-opaque substance in an amount effective to render the marker imageable in a computed tomographic scan.

**12.** The device claim 11, **characterised in that** the radio-opaque substance comprises a compound containing an atom having a high atomic number.

**13.** The device of claim 12, **characterised in that** the atom is selected from the group consisting of barium, titanium, silver, gold and platinum.

**14.** The device of claim 13, **characterised in that** the radiopaque substance is provided as a salt.

**15.** The device of claim 13, **characterised in that** the salt is provided in a concentration of up to about 400mg/ml.

**16.** The device of claim 1, **characterised in that** the first solution contains a compound containing an atom having a high atomic number.

**17.** The device of claim 16, **characterised in that** said atom is selected from the group of elements consisting of, barium, iodine, titanium, gold, tantalum, silver, platinum and iron.

**18.** The device of claims 16 or 17, **characterised in that** the concentration of the element provided in the first aqueous solution is present in an amount effective to render the volume filled by the solution radio-opaque.

**19.** The device of claim 18, **characterised in that** the first solution contains (a) iohezol or, (b) silver nitrate or, (c) iothalamate meglumine.

**20.** The device of claim 19, **characterised in that** the concentration of silver nitrate is about 350mg/ml.

**21.** The device of claim 19, **characterised in that** the concentration of silver nitrate is about 200mg/ml, and wherein the aqueous solution contains gadopentetate dimeglumine-DPTA at a concentration of about 0.5mM.

**22.** The device of claim 19, **characterised in that** the concentration of iothalamate meglumine is about 175mg of iodine/ml, and wherein the second aqueous solution contains gadopentetate dimeglumine-DPTA at a concentration of about 0.5mM.

**23.** The device of claim 19, **characterised in that** the concentration of iothalamate meglumine is about 165mg of iodine/ml.

**24.** The device of claim 1, **characterised in that** the second solution contains gadopentetate dimeglumine-DPTA.

**25.** The device of claim 24, **characterised in that** the gadopentetate dimeglumine-DPTA is provided at a concentration of about 0.5mM.

**26.** The device of claim 1, **characterised in that** the first solution is an aqueous solution of silver nitrate and the second solution is an aqueous solution of gadopentetate dimeglumine-DPTA.

**27.** The device of claim 26, **characterised in that** the concentration of the silver nitrate is about 350mg/ml, and the concentration of the gadopentetate dimeglumine-DPTA is about 0.5mM.

**28.** The device of claim 1, **characterised in that** the first solution is an aqueous solution of iothalamate meglumine and the second solution is an aqueous solution of gadopentetate dimeglumine-DPTA.

**29.** The device of claim 28, **characterised in that** the concentration of the iothalamate meglumine is about 175mg of iodine/ml, and the concentration of the gadopentetate dimeglumine-DPTA is about 0.5mM.

**30.** The device of claim 1, **characterised in that** the fiducial marker assembly is sized so as to permit the long-term implantation of the assembly into a patient without causing the distortion of the tissue during the period during which the device is implanted.

**31.** A method for providing a fiducial marker according to claim 1 that is imageable under computerised X-ray tomography and nuclear magnetic resonance imaging, comprising the steps of: providing the cavity with a first aqueous solution which is radiopaque and thus imageable under computerised X-ray tomography, and mixing the first solution with a second aqueous imaging agent that is imageable under nuclear magnetic resonance imaging, wherein the centres of the regions of the fiducial marker that are defined by each imaging agent are coincident, thereby permitting registration of images obtained by each imaging modality.

**Patentansprüche**

**1.** Bezugspunktmarkiereinrichtungsanordnung, einschließend:

eine Abbildungsmarkiereinrichtung (10), umfassend Abbildungsmaterial und aufweisend ein aus einem biokompatiblen Material konstruiertes Gehäuse, wobei die Abbildungsmarkiereinrichtung (10) einen Hohlraum (14) aufweist, **dadurch gekennzeichnet, dass** der Hohlraum (14) eine Mischung von wässrigen Abbildungsmaterialien enthält, die es ermöglichen, dass die Abbildungsmarkiereinrichtung (10) unter Röntgenstrahlen, CT und MRI abbildbar ist, wobei die Abbildungsmaterialien eine erste und eine zweite wässrige Lösung sind, die mischbar sind, um innerhalb des Hohlraums (14) eine Mischung zu bilden, wobei die erste Lösung strahlenundurchlässig ist und die zweite Lösung unter magnetischer Kernresonanz abbildbar ist.

2. vorrichtung nach Anspruch 1, **dadurch gekennzeichnet, dass** sie weiter umfasst:

eine Basis (30) zum Halten der Abbildungsmarkiereinrichtung (10), wobei die Basis (30) eine Einrichtung aufweist, um der Anbringung der Basis (30) an Gewebe Rechnung zu tragen; einen Verbinder zum abnehmbaren Befestigen der Abbildungsmarkiereinrichtung (10) an der Basis (30); und eine Einrichtung zum Abdichten des Hohlraums (14) der Abbildungsmarkiereinrichtung (10) gegen Entweichen von im Hohlraum (14) eingeschlossenem Abbildungsmaterial.

3. Vorrichtung nach Anspruch 2, **dadurch gekennzeichnet, dass** die Einrichtung zum Abdichten (24) des Hohlraums (14) ein Deckel (16) ist, der sicher mit dem Gehäuse der Abbildungsmarkiereinrichtung (10) verbunden ist.

4. Vorrichtung nach Anspruch 3, **dadurch gekennzeichnet, dass** der Deckel (16) einen überstehenden Vorsprung (20) einschließt, und die Basis eine entsprechende Buchse (36, 38) einschließt, die so bemessen ist, dass sich der Vorsprung (20) des Deckels (16) mit der Buchse (36, 38) paaren und **dadurch** eine Verbindung zwischen dem Deckel (16) und der Basis (30) bilden kann.

5. Vorrichtung nach den Ansprüchen 2, 3 oder 4, **dadurch gekennzeichnet, dass** die Abdichteinrichtung (24) mit einer Mehrzahl von ausgestreckten Armen (18) zur abnehmbaren Anbringung an der Basis (30) versehen ist.

6. Vorrichtung nach den Ansprüchen 2, 4 oder 5, **dadurch gekennzeichnet, dass** die Basis (30) ein Gewinde (32) einschließt, um ihre Anbringung an Gewebe zu erleichtern.

7. Vorrichtung nach Anspruch 5, **dadurch gekennzeichnet, dass** die Basis (40) mit Nuten (34) versehen ist, und die ausgestreckten Arme (18) der Abdichteinrichtung (24) in vorstehenden Teilen (22) enden, die mit den Nuten (34) auf der Basis zur Verbindung damit zusammenwirken.

8. Vorrichtung nach einem der vorangehenden Ansprüche, **dadurch gekennzeichnet, dass** das Markiereinrichtungsgehäuse so geformt ist, dass sein Volumenschwerpunkt mit dem Volumenschwerpunkt des darin begrenzten Hohlraums zusammenfällt.

9. Vorrichtung nach Anspruch 8, **dadurch gekennzeichnet, dass** das Markiereinrichtungsgehäuse zylindrisch geformt ist.

10. Vorrichtung nach den Ansprüchen B oder 9, **dadurch gekennzeichnet, dass** das Markiereinrichtungsgehäuse aus einem Polymer gebildet ist.

11. Vorrichtung nach Anspruch 1, **dadurch gekennzeichnet, dass** das Markiereinrichtungsgehäuse mit einer strahlenundurchlässigen Substanz versetzt ist, und zwar in einer Menge, die wirksam ist, um die Markiereinrichtung in einer computertomographischen Abtastung abbildbar zu machen.

12. Vorrichtung nach Anspruch 11, **dadurch gekennzeichnet, dass** die strahlenundurchlässige Substanz eine Verbindung umfasst, die ein Atom mit einer hohen Atomzahl enthält.

13. Vorrichtung nach Anspruch 12, **dadurch gekennzeichnet, dass** das Atom aus der aus Barium, Titan, Silber, Gold und Platin bestehenden Gruppe ausgewählt ist.

14. Vorrichtung nach Anspruch 13, **dadurch gekennzeichnet, dass** die strahlenundurchlässige Substanz als Salz vorgesehen ist.

15. Vorrichtung nach Anspruch 13, **dadurch gekennzeichnet, dass** das Salz in einer Konzentration von bis zu etwa 400 mg/ml vorgesehen ist.

16. Vorrichtung nach Anspruch 1, **dadurch gekennzeichnet, dass** die erste Lösung eine Verbindung enthält, die ein Atom mit einer hohen Atomzahl enthält.

17. Vorrichtung nach Anspruch 16, **dadurch gekennzeichnet, dass** das Atom aus der Gruppe von Elementen bestehend aus Barium, Jod, Titan, Gold, Tantal, Silber, Platin und Eisen ausgewählt ist.

18. Vorrichtung nach den Ansprüchen 16 oder 17, **dadurch gekennzeichnet, dass** die Konzentration des in der ersten wässrigen Lösung vorgesehenen Elements in einer Menge vorhanden ist, die wirksam ist, um das mit der Lösung gefüllte Volumen strahlenundurchlässig zu machen.

19. Vorrichtung nach Anspruch 18, **dadurch gekennzeichnet, dass** die erste Lösung (a) Iohexol oder (b) Silbernitrat oder (c) Iothalamat-Meglumin enthält.

20. Vorrichtung nach Anspruch 19, **dadurch gekennzeichnet, dass** die Silbernitrat-Konzentration etwa 350 mg/ml beträgt.

21. Vorrichtung nach Anspruch 19, **dadurch gekennzeichnet, dass** die Silbernitrat-Konzentration etwa 200 mg/ml beträgt, und in der die wässrige Lösung Gadopentetat-Dimeglumin-DPTA in einer Konzentration von etwa 0,5 mM enthält.

22. Vorrichtung nach Anspruch 19, **dadurch gekennzeichnet, dass** die Iothalamat-Meglumin-Konzentration etwa 175 mg Jod/ml beträgt, und in der die zweite wässrige Lösung Gadopentetat-Dimeglumin-DPTA in einer Konzentration von etwa 0.5 mM enthält.

23. Vorrichtung nach Anspruch 19, **dadurch gekennzeichnet, dass** die Iothalamat-Meglumin-Konzentration etwa 165 mg Jod/ml beträgt.

24. Vorrichtung nach Anspruch 1, **dadurch gekennzeichnet, dass** die zweite Lösung Gadopentetat-Dimeglumin-DPTA enthält.

25. Vorrichtung nach Anspruch 24, **dadurch gekennzeichnet, dass** das Gadopentetat-Dimeglumin-DPTA in einer Konzentration von etwa 0,5 mM vorgesehen ist.

26. Vorrichtung nach Anspruch 1, **dadurch gekennzeichnet, dass** die erste Lösung eine wässrige Silbernitrat-Lösung ist, und die zweite Lösung eine wässrige Gadopentetat-Dimeglumin-DPTA-Lösung ist.

27. Vorrichtung nach Anspruch 26, **dadurch gekennzeichnet, dass** die Silbernitrat-Konzentration etwa 350 mg/ml beträgt, und die Konzentration des Gadopentetat-Dimeglumin-DPTA etwa 0,5 mM beträgt.

28. Vorrichtung nach Anspruch 1, **dadurch gekennzeichnet, dass** die erste Lösung eine wässrige Iothalamat-Meglumin-Lösung ist, und die zweite Lösung eine wässrige Gadopentetat-Dimeglumin-DPTA-Lösung ist.

29. Vorrichtung nach Anspruch 28, **dadurch gekennzeichnet, dass** die Konzentration des zothalamat-Meglumins etwa 175 mg Jod/ml beträgt, und die Konzentration des Gadopentetat-Dimeglumin-DPTA etwa 0,5 mM beträgt.

30. Vorrichtung nach Anspruch 1, **dadurch gekennzeichnet, dass** die Bezugspunkt-Markiereinrichtungsanordnung so bemessen ist, dass sie die Langzeitimplantation der Anordnung in einen Patienten gestattet, ohne während des Zeitraums, während dem Vorrichtung implantiert ist, die verformung des Gewebes zu verursachen.

31. Verfahren zur Bereitstellung einer Bezugspunkt-Markiereinrichtung nach Anspruch 1, die unter rechnergesteuerter Röntgen-Tomographie und magnetischer Kernresonanz-Abbildung abbildbar ist, umfassend die Schritte: Versehen des Hohlraums mit einer ersten wässrigen Lösung, die strahlenundurchlässig ist und somit unter rechnergesteuerter Röntgen-Tomographie abbildbar ist, und Mischen der ersten Lösung mit einem zweiten wässrigen Abbildungsmittel, das unter magnetischer Kernresonanz-Abbildung abbildbar ist, wobei die Mitten der Bereiche der Bezugspunkt-Markiereinrichtung, die von jedem Abbildungsmittel festgelegt werden, zusammenfallen, wodurch eine passgenaue Deckung von durch jede Abbildungsweise erhaltenen Bildern gestattet wird.

## Revendications

1. Ensemble de marqueur de calibrage, comportant :

   un marqueur d'imagerie (10) comprenant un matériau d'imagerie et ayant un boîtier réalisé dans un matériau biocompatible, le marqueur d'imagerie (10) ayant une cavité (14) **caractérisé en ce que** la cavité (14) contient un mélange de matériaux d'imagerie aqueux qui permettent au marqueur d'imagerie (10) de pouvoir être transformé en image sous imagerie par résonance magnétique ou tomodensitométrie aux rayons X ; les matériaux d'imagerie étant des première et seconde solutions aqueuses qui sont miscibles pour former un mélange à l'intérieur de la cavité (14) dans laquelle la première solution est opaque aux radiations et la seconde solution peut être transformée en image sous résonance magnétique nucléaire.

2. Dispositif selon la revendication 1, **caractérisé en ce qu'**il comprend en outre :

   une base (30) permettant de supporter le marqueur d'imagerie (10), ladite base (30) ayant des moyens permettant la fixation de la base (30) sur des tissus ;
   un raccord permettant de fixer, de manière amovible, le marqueur d'imagerie (10) à la base (30) ; et
   un moyen permettant d'étanchéifier la cavité (14) du marqueur d'imagerie (10) contre les fuites du matériau d'imagerie contenu dans la cavité (14).

3. Dispositif selon la revendication 2, **caractérisé en ce que** le moyen d'étanchéité (24) de la cavité (14) est un bouchon (16) qui est raccordé de manière fixe au boîtier du marqueur d'imagerie (10).

4. Dispositif selon la revendication 3, **caractérisé en ce que** le bouchon (16) comporte un bossage faisant saillie (20) et **en ce que** la base comporte une emboîture correspondante (36, 38) ayant des dimensions telles que le bossage (20) du bouchon (16) peut correspondre à l'emboîture (36, 38) et ainsi former un lien entre le bouchon (16) et la base (30).

5. Dispositif selon les revendications 2, 3 ou 4, **caractérisé en ce que** le moyen d'étanchéité (24) est muni d'une pluralité de bras extensibles (18) permettant de le fixer, de manière amovible, à la base (30).

6. Dispositif selon les revendications 2, 4 ou 5, **caractérisé en ce que** la base (30) inclut un filetage (32) permettant de faciliter la fixation au tissu.

7. Dispositif selon la revendication 5, **caractérisé en ce que** la base (40) est munie de rainures (34) et **en ce que** les bras extensibles (18) du moyen d'étanchéité (24) s'achèvent en des parties faisant saillie (22) qui coopèrent avec les rainures (34) sur la base pour permettre le raccord avec celle-ci.

8. Dispositif selon l'une quelconque des revendications précédentes, **caractérisé en ce que** le boîtier du marqueur est d'une forme telle que le centroïde du volume coïncide avec le centroïde du volume de la cavité définie dans celui-ci.

9. Dispositif selon la revendication 8, **caractérisé en ce que** le boîtier du marqueur est de forme cylindrique.

10. Dispositif selon la revendication 8 ou 9, **caractérisé en ce que** le boîtier du marqueur est formé d'un polymère.

11. Dispositif selon la revendication 1, **caractérisé en ce que** le boîtier du marqueur est dopé avec une substance opaque aux radiations en une quantité suffisante pour rendre le marqueur susceptible d'être transformé en image dans un tomodensitomètre.

12. Dispositif selon la revendication 11, **caractérisé en ce que** la substance opaque aux radiations comprend un composé contenant un atome ayant un numéro atomique élevé.

13. Dispositif selon la revendication 12, **caractérisé en ce que** l'atome est choisi dans le groupe consistant en le baryum, le titane, l'argent, l'or et le platine.

14. Dispositif selon la revendication 13, **caractérisé en ce que** la substance opaque aux radiations est fournie en tant qu'un sel.

15. Dispositif selon la revendication 13, **caractérisé en ce que** le sel est fourni en une concentration allant jusqu'à environ 400 mg/ml.

16. Dispositif selon la revendication 1, **caractérisé en ce que** la première solution contient un composé contenant un atome ayant un numéro atomique élevé.

17. Dispositif selon la revendication 16, **caractérisé en ce que** l'atome est choisi dans le groupe d'éléments consistant en le baryum, l'iode, le titane, l'or, le tantale, l'argent, le platine et le fer.

18. Dispositif selon la revendication 16 ou 17, **caractérisé en ce que** la concentration de l'élément fourni dans la première solution aqueuse est présente en une quantité suffisante pour remplir le volume avec la solution opaque aux radiations.

19. Dispositif selon la revendication 18, **caractérisée en ce que** la première solution contient (a) de l'iohézole ou, (b) du nitrate d'argent ou, (c) de l'iothamalate de méglumine.

20. Dispositif selon la revendication 19, **caractérisé en ce que** la concentration en nitrate d'argent est d'environ 350 mg/ml.

21. Dispositif selon la revendication 19, **caractérisé en ce que** la concentration en nitrate d'argent est d'environ 200 mg/ml, et dans lequel la solution aqueuse contient du gadopentetate de diméglumine - DPTA en une concentration d'environ 0,5 mM.

22. Dispositif selon la revendication 19, **caractérisé en ce que** la concentration d'iothamalate de méglumine est d'environ 175 mg d'iode/ml, et dans lequel la seconde solution aqueuse contient du gadopentetate de diméglumine - DPTA en une concentration d'environ 0,5 mM.

23. Dispositif selon la revendication 19, **caractérisé en ce que** la concentration en iothamalate de méglumine est d'environ 165 mg d'iode/ml.

24. Dispositif selon la revendication 1, **caractérisé en ce que** la seconde solution contient du gadopentetate de diméglumine - DPTA.

**25.** Dispositif selon la revendication 24, **caractérisé en ce que** le gadopentetate de diméglumine - DPTA est fourni en une concentration d'environ 0,5 mM.

**26.** Dispositif selon la revendication 1, **caractérisé en ce que** la première solution est une solution aqueuse de nitrate d'argent et la seconde solution est une solution aqueuse de gadopentetate de diméglumine - DPTA.

**27.** Dispositif selon la revendication 26, **caractérisé en ce que** la concentration en nitrate d'argent est d'environ 350 mg/ml, et la concentration en gadopentetate de diméglumine - DPTA est d'environ 0,5 mM.

**28.** Dispositif selon la revendication 1, **caractérisé en ce que** la première solution est une solution aqueuse d'iothamalate de méglumine et la seconde solution est une solution aqueuse de gadopentetate de diméglumine - DPTA.

**29.** Dispositif selon la revendication 28, **caractérisé en ce que** la concentration en iothamalate de méglumine est d'environ 175 mg d'iode/ml, et la concentration en gadopentetate de diméglumine - DPTA est d'environ 0,5 mM.

**30.** Dispositif selon la revendication 1, **caractérisé en ce que** l'ensemble de marqueur de calibrage a des dimensions permettant une implantation à long terme dans un patient sans provoquer de déformation des tissus au cours de la période pendant laquelle le dispositif est implanté.

**31.** Procédé permettant de fournir un marqueur de calibrage selon la revendication 1 qui peut être transformé en image sous imagerie par résonance magnétique nucléaire ou tomographie aux rayons X, comprenant les étapes consistant à : fournir à la cavité une première solution aqueuse qui est opaque aux radiations et ainsi peut être transformé en image sous tomodensitométrie aux rayons X, et mélanger la première solution aqueuse avec un second agent d'imagerie aqueux qui peut être transformé en image sous imagerie par résonance magnétique nucléaire, dans lequel les centres des régions du marqueur de calibrage qui sont définis par chaque agent d'imagerie coïncident, permettant ainsi un enregistrement des images obtenues par chaque technique d'imagerie.

FIG. 1A

FIG. 1B

FIG. 2B

FIG. 2A

FIG.3A

FIG.3B

FIG.3C

FIG. 4B

FIG. 4A

FIG. 4C

FIG. 5

EP 1 249 207 B1